# EUROPEAN PATENT SPECIFICATION

(11) **EP 4 385 023 B1**
(45) Date of publication and mention of the grant of the patent: **16.07.2025**
(21) Application number: 22764754.2
(22) Date of filing: 12.08.2022
(51) Int. Cl.: G16B 30/00, G16B 40/00, G16B 40/20

(54) **MEANS AND METHODS FOR RESISTANCE PREDICTION TO ANTI-INFECTIVES**
MITTEL UND VERFAHREN ZUR RESISTENZVORHERSAGE GEGEN ANTIINFEKTIVA
MOYENS ET PROCÉDÉS DE PRÉDICTION DE LA RÉSISTANCE AUX ANTI-INFECTIEUX

(30) Priority: 13.08.2021 EP 21191251; 22.09.2021 EP 21198248
(43) Date of publication of application: 19.06.2024
(73) Proprietor: Noscendo GmbH, 47198 Duisburg (DE)
(72) Inventor: STEVENS, Philip, 46519 Alpen (DE); CLANZETT, Sebastian, 46519 Alpen (DE); BURGSTALLER-MÜHLBACHER, Sebastian, 1070 Wien (AT); VON HAESELER, Arndt, 2403 Scharndorf (AT)
(74) Representative: Schiweck Weinzierl Koch Patentanwälte Partnerschaft mbB
(86) International application number: PCT/EP2022/072686
(87) International publication number: WO 2023/017156

(56) References cited:
- ANAHTAR MELIS N. ET AL: "Applications of Machine Learning to the Problem of Antimicrobial Resistance: an Emerging Model for Translational Research", JOURNAL OF CLINICAL MICROBIOLOGY, vol. 59, no. 7, 18 June 2021 (2021-06-18), US, XP055888891, ISSN: 0095-1137, DOI: 10.1128/JCM.01260-20
- VAN CAMP PIETER-JAN ET AL: "Prediction of Antimicrobial Resistance in Gram-Negative Bacteria From Whole-Genome Sequencing Data", FRONTIERS IN MICROBIOLOGY, vol. 11, 25 May 2020 (2020-05-25), XP055888908, DOI: 10.3389/fmicb.2020.01013
- VAN CAMP PIETER-JAN ET AL: "Bioinformatics Approaches to the Understanding of Molecular Mechanisms in Antimicrobial Resistance", INTERNATIONAL JOURNAL OF MOLECULAR SCIENCES, vol. 21, no. 4, 18 February 2020 (2020-02-18), pages 1363, XP055888939, DOI: 10.3390/ijms21041363
- MARCUS D BLOICE ET AL: "Augmentor: An Image Augmentation Library for Machine Learning", THE JOURNAL OF OPEN SOURCE SOFTWARE, vol. 2, no. 19, 11 August 2017 (2017-08-11), pages 432, XP055561652, DOI: 10.21105/joss.00432

## Description

The present invention relates to a computer-implemented method for classifying sensitivities or resistances, respectively, of a sample pathogen against one or more anti-infectives from biological sequence data of said sample pathogen. Also provided is a method for augmenting sequence data. Furthermore, the present invention relates to a computer program product adapted to carry out the method.

The global emergence of resistance of pathogens against anti-infectives, together with the lack and reduced development of new antibiotic molecules, currently poses a serious public health problem, as it may mean the return to a pre-antibiotic era in which infections caused by multi-resistant pathogens are persistent.

Antibiotic resistance happens when germs like bacteria and fungi develop the ability to defeat the drugs designed to kill them. That means the germs are not killed and continue to grow. Infections caused by antibiotic-resistant germs are difficult, and sometimes impossible, to treat. In most cases, antibiotic-resistant infections require extended hospital stays, additional follow-up doctor visits, and costly and toxic alternatives. Antibiotic resistance has the potential to affect people at any stage of life, as well as the healthcare, veterinary, and agriculture industries, making it one of the world's most urgent public health problems. Presently, when a human subject, such as a patient may suffer from sepsis caused by an unknown pathogen, usually blood is drawn with the aim of culturing the pathogen to then identify and set up an antibiogram of said pathogen in order to get a clue against which anti-infective the pathogen may or may not be resistant. Since this procedure is time consuming, while in essence no time should be lost, patients are oftentimes treated with a so called broad-spectrum antibiotic or sometimes with a few antibiotics which aim at different targets within the pathogen. Yet, this "shot-gun" approach although it is feasible to do with the aim of treating a subject, e.g. a patient as soon and as effective as possible, it would be beneficial to such patients if as less as possible time is lost until start of treatment and, all the more, hit the pathogen with an antibiotic against which it is not resistant.

From the very beginning, the interest of Institutes of Microbiology, Hygiene and Public Health and of Infectious Diseases have focused on antibiotic resistance: from molecular mechanisms to epidemiology, clinical issues and prevention. The current standard to identify and diagnose anti-infective resistances includes the need to cultivate the germ or pathogen under investigation.

Next generation sequencing (NGS) allows in a short period of time the generation of sequencing data, e.g. from pathogens. Based on sequencing data it may be possible to allocate a resistance to an anti-infective to, e.g. a specific allele of a pathogen. However, the task gets more complicated if the pathogen is not known, cannot be determined, etc. In other words, no reliable means and methods are available which would allow to predict whether or not an unknown pathogen present in a subject may be resistant against one or more anti-infectives. Indeed, a reliable statement from data not obtained from isolates has not yet been possible because either phenotyping methods have been used or a selection has taken place as to which genomic regions have been used for the detection/prediction of antimicrobial resistance.

In the field of machine learning, or in a broader context, artificial intelligence (AI) this often leads to a high sensitivity/specificity for single combinations of germs and agents but it does not solve the problem of providing a "universal" prediction method.

Likewise, areas of the respective pathogen are often "learned" which contain special characteristics, e.g. single mutations (SNPs), in order to detect a difference between the native wild type (antibiotics (AB) sensitive) and the resistant type. However, especially in data from native material of a subject, e.g. patient, e.g. blood, urine, liquor etc., not all genomic regions of a germ are present/sequenced with high probability. Therefore, a prediction/detection of resistance using such "marker sequences" is not always, or only rarely, possible.

Thus, further means and methods are needed that may be employed in the detection of resistance (or, vice versa, sensitivity) of pathogens to anti-infectives. It is highly desired to satisfy this need.

The document ANAHTAR MELIS N. ET AL: "Applications of Machine Learning to the Problem of Antimicrobial Resistance: an Emerging Model for Translational Research" is a scientific publication relating to the use of machine learning techniques for solving the problem of predicting antimicrobial resistances. This document acknowledges the small size of the training set as a shortcoming without offering a solution.

Hence, the present invention addresses this need and provides a solution thereto as described herein, defined in the claims, illustrated in the examples and shown in the figures.

Accordingly, the present invention provides a computer-implemented method for classifying resistances of a sample pathogen against one or more anti-infectives from biological sequence data of said sample pathogen, the method comprising:
augmenting sequence data of a first plurality of biological sequences of pathogens, different from the sample pathogen, having known resistances against the one or more anti-infectives, to sequence data of a second plurality of variant biological sequences by generating sequence data of multiple variants of each sequence of the first plurality of sequences, each variant carrying the same resistances against the one or more anti-infectives as the corresponding sequence of the first plurality of sequences, wherein
generating sequence data of multiple variants for each sequence of the first plurality of sequences, referred to as first sequence comprises:
   partitioning the first sequence into multiple parts, each part not exceeding predetermined length; and
   generating multiple variants of the first sequence by concatenating at most once randomly selected parts of said multiple parts to respective variants of the first sequence, thereby obtaining sequence data of the second plurality of biological sequences;
   training a neural network, NN, via a input layer, specimen sequence data of the second plurality of biological sequences of pathogens labelled with known resistances of the respective pathogens, using forward propagation that repeatedly compares outputs of the neural network with corresponding ground truth labels to calculate an error using a loss function, and using backpropagation to calculate a gradient of the loss function to adjust the weighting values of the NN,
   obtaining, after training the NN, biological sequence data for inferring resistances of a sample pathogen; and
   passing said biological sequence data to an input layer of the NN with weights and biases after teaching incorporated;
   generating, via an inference output layer of the NN, classification predictions of resistances of the sample pathogen against the one or more anti-infectives obtained from output of the neural network.

In the alternative to methods for classifying resistances of a sample pathogen against one or more anti-infectives from biological sequence data, the present invention also relates to methods for classifying sensitivities of a sample pathogen against one or more anti-infective from biological sequence data.

Thus, whenever herein "resistances" is disclosed or is present in the context of the present invention, in particular in the context of the methods for classifying resistances of a sample pathogen against one or more anti-infectives from biological sequence data, it is noted that it can be replaced by "sensitivities". Indeed, the computer- implemented method of the present invention allows for classifying either resistances or sensitivities of a sample pathogen against one or more anti-infectives. It is merely a question of what a skilled person is looking for, i.e., either resistances or sensitivities, since the neural network can be trained either way.

Accordingly, disclosure, such as embodiments, preferred embodiments, etc. disclosed in the context of methods for classifying resistances of a sample pathogen against one or more anti-infectives from biological sequence data, also pertain, mutatis mutandis, to methods for classifying sensitivities of a sample pathogen against one or more anti-infectives from biological sequence data

Accordingly, in the alternative, the present invention provides a computer-implemented method for classifying sensitivities of a sample pathogen against one or more anti-infectives from biological sequence data of said sample pathogen, the method comprising:
augmenting sequence data of a first plurality of biological sequences of pathogens, different from the sample pathogen, having known sensitivities against the one or more anti-infectives, to sequence data of a second plurality of variant biological sequences by generating sequence data of multiple variants of each sequence of the first plurality of sequences, each variant carrying the same sensitivities against the one or more anti-infectives as the corresponding sequence of the first plurality of sequences, wherein
generating sequence data of multiple variants for each sequence of the first plurality of sequences, referred to as first sequence comprises:
   partitioning the first sequence into multiple parts, each part not exceeding predetermined length; and
   generating multiple variants of the first sequence by concatenating at most once randomly selected parts of said multiple parts to respective variants of the first sequence, thereby obtaining sequence data of the second plurality of biological sequences;
   training a neural network, NN, via a input layer, specimen sequence data of the second plurality of biological sequences of pathogens labelled with known sensitivities of the respective pathogens, using forward propagation that repeatedly compares outputs of the neural network with corresponding ground truth labels to calculate an error using a loss function, and using backpropagation to calculate a gradient of the loss function to adjust the weighting values of the NN,
   obtaining, after training the NN, biological sequence data for inferring sensitivities of a sample pathogen; and
   passing said biological sequence data to an input layer of the NN with weights and biases after teaching incorporated;
generating, via an inference output layer of the NN, classification predictions of sensitivities of the sample pathogen against the one or more anti-infectives obtained from output of the neural network.

Concerning the variants of each sequence of the first plurality of sequences, it is irrelevant whether they comprise the known marker sequences for the resistances against the anti-infectives. Even if those marker sequences are absent in the variants, the remaining sequence information comprises enough potentially undescribed effects/dependencies/correlations, which may be responsible for those resistances in an indirect manner. By varying the position and the presence of the multiple parts in the different variants, the neural network is trained with a large number of different variants which all comprise patterns being indirectly or even directly linked to the the resistance or sensitivity to the anti-infective. Thus, the neural network "learns" to recognize the complete pattern of the first plurality of sequences of the pathogens having known resistances against one or more anti-infectives. Hence, the neural network does not "learn" to recognize the known marker sequences in the variants for those resistances but "learns" to recognize the context in which those marker sequences occur, i.e. on the sequence level (i.e. available sequence information).

Thus, thanks to the means and methods of the present invention, it is neither necessary to know the pathogen nor is it necessary to have available a fully sequenced genome of a pathogen nor is it necessary to check the genome or parts thereof of a pathogen for one or the other single nucleotide polymorphism which may be known to be linked with resistance against an anti-infective, etc. The computer-implemented methods of the present invention, hereinafter also simply referred to as "method" or "methods" of the present invention make preferably use of deep learning algorithms, e.g. as described in LeCun ez al, Nature 521, 436-444 (2015): http://dx.doi.org/10.1038/nature14539.

It is only required to have a sample, e.g. from a subject, such as a patient, which contains nucleic acids which can be sequenced and subsequently analyzed by applying the method and means of the present invention.

Thus, after training the neural network with the augmented data, the neural network may provide classification predictions of resistances of the sample against the one or more anti-infectives. Furthermore, it is sufficient to provide samples from native material of a subject, e.g. patient, e.g. blood, urine, liquor, in which not all genomic regions of a germ are present/sequenced with high probability. Since the neural network trained according to the invention recognizes the whole pattern of the available genomic regions, the presence or absence of marker sequences will not affect the classification predictions concerning the resistance of the pathogen.

Both complete genome sequences of anti-infective resistant and anti-infective sensitive genomes may be used to train the network applied in the methods of the present invention. Thus, ideally, the neural network learns the difference between sensitive and resistant pathogens on the sequence level (i.e. available sequence information). For this purpose, either the actual sequences, k-mer frequencies or similar parameters can be used.

These differences, in comparison to prior art methods, allow the neural network, preferably a CNN, to use the direct and indirect differences in the available sequence information to detect/predict resistance rather than learning individual elements, e.g. single point mutations.

Furthermore, the entire genetic information is preferably used and not only marker sequences. This leads to the fact that potentially undescribed effects/dependencies/correlations within a genome, which are not directly responsible for resistance, can detect/predict resistance.

According to the coverage in the training dataset/database, a variety of resistances or sensitivities, respectively, can be found/predicted from a single sample. Moreover, the means and methods of the present invention are able to detect combinations/mixtures of resistances or sensitivities, respectively. While many tools infer resistance from the determination of the pathogen species, the means and methods of the present invention infer the resistance conferring information directly.

This decoupling of resistance and species, implies that the corresponding characteristics can be applied in an irreversible way and thus a lateral transmission of resistances that has not been described before can be detected.

That said, another important advantage of the means and methods of the present invention is the combination of two properties: by the quantitative measurement and the probabilistic description of a possibly unnatural accumulation of signals for a certain resistance mediating mechanism in one measurement, it is possible for the first time to use detected signals of different species within a subject, e.g. a patient and thus provide information that contributes to the decision on treatment relevance.

Due to the highly sensitive and specific diagnosis, it is possible to initiate pathogen-specific treatment in a short time. A major advantage of this is the supposedly resulting decrease in multi-resistant pathogens through resistancespecific anti-infective therapy.

The means and methods of the present invention bring thus benefit to the field of diagnosing and (indirectly) treating infectious diseases in the healthcare setting for, e.g. "inpatients" as well as "out-patients".

Of note, no special or proprietary data for training is required. Rather, available genomes or parts thereof of pathogens can be used, genomes of newly isolated pathogens can be used, etc. Ideally, it is known whether available genomes of pathogens or parts thereof may be linked with resistance to one or more anti-infectives. Similarly, ideally, it is known or checked whether genomes of newly isolated pathogens may be linked with resistance to one or more anti-infectives.

The present inventors found that the way they used data augmentation allowed them to train the neural network to then achieve the classification of resistances or sensitivities, respectively, of a sample pathogen against one or more anti-infectives from biological sequence data of said sample pathogen.

Training of the neural network, preferably a CNN is, e.g. based on a database of entire or partial genomes of anti-infective resistant and anti-infective sensitive pathogens. The genomes in this database may need to either be pre-processed by subsampled fragments of fixed, arbitrary length of the original genome or transcriptome or the entire genomes or transcriptomes can be used as an input to the neural network, preferably a CNN.

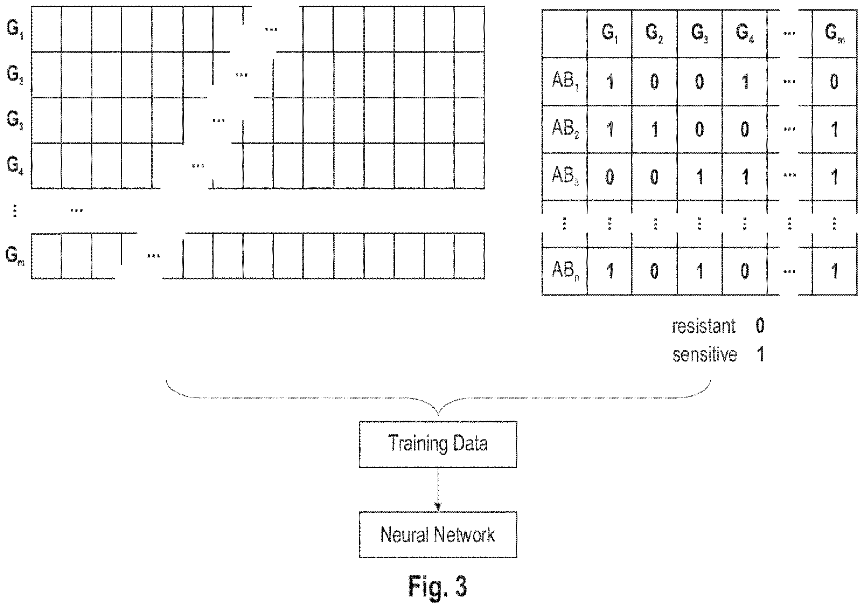

Figure 3 shows a preferred training of a neural network.

The term "organism" when referred to herein encompasses a subject and a pathogen, respectively, or a part thereof, e.g. nucleic acids or proteins or both, as described herein.

The term "pathogen" when used herein encompasses a microorganism, such as a virus, a bacterium, a fungus or a parasite.

The term "subject" when used herein encompasses a vertebrate, preferably a mammal, for example, human, dog, cat, pig, horse, cattle, sheep, goat, mouse, or rat. Preferably the subject is a human, e.g. a patient, such as a human patient. The subject may contain a pathogen, e.g. it may suffer from a disease caused by a pathogen.

Although, in the methods of the present invention it is neither necessary nor foreseen to identify the pathogen so as to determine, e.g., the species, it is envisioned that the following pathogens may be among the pathogens from which the methods of the present invention classify resistances or sensitivities, respectively, to one or more anti-infectives.

Exemplary bacteria include, but are not limited to, Neisseria meningitis Streptococcus pneumoniae, Streptococcus pyogenes, Moraxella catarrhalis, Bordetella pertussis, Staphylococcus aureus, Clostridium tetani, Corynebacterium diphtheria, Haemophilus influenza, Pseudomonas aeruginosa, Streptococcus agalactiae, Chlamydia trachomatis, Chlamydia pneumoniae, Helicobacter pylori, Escherichia coli, Bacillus anthracis, Yersinia pestis, Staphylococcus epidermis, Clostridium perfringens, Clostridium botulinum, Legionella pneumophila, Coxiella burnetii, Brucella spp. such as B. abortus, B. canis, B. melitensis, B. neotomae, B. ovis, B. suis, B. pinnipediae, Francisella spp. such as F. novicida, F. philomiragia, F. tularensis, Neisseria gonorrhoeae, Treponema pallidum, Haemophilus ducreyi, Enterococcus faecalis, Enterococcus faecium, Staphylococcus saprophyticus, Yersinia enterocolitica, Mycobacterium tuberculosis, Rickettsia spp., Listeria monocytogenes, Vibrio cholera, Salmonella typhi, Borrelia burgdorferi, Porphyromonas gingivalis, Klebsiella spp., Klebsiella pneumoniae.

Exemplary viruses include, but are not limited to, Orthomyxoviridae, such as influenza A, B or C virus; Paramyxoviridae viruses, such as Pneumoviruses (e.g., respiratory syncytial virus, PvSV), Rubulaviruses (e.g., mumps virus), Paramyxoviruses (e.g., parainfluenza virus), Metapneumoviruses and Morbilliviruses (e.g., measles); Poxviridae, such as Orthopoxvirus (e.g., Variola vera, including Variola major and Variola minor); Picornaviridae, such as Enteroviruses (e.g., poliovirus e.g. a type 1 , type 2 and/or type 3 poliovirus, EV71 enterovirus, coxsackie A or B virus), Rhinoviruses, Heparnavirus, Cardioviruses and Aphtho viruses; Bunyaviruses, such as Orthobunyavirus (e.g., California encephalitis virus), Phlebovirus (e.g., Rift Valley Fever virus), or Neurovirus (e.g., Crimean-Congo hemorrhagic fever virus); Heparnaviruses (e.g., hepatitis A virus (HAV), B and C); Filoviridae (e.g., Ebola virus (including a Zaire, ivory Coast, Reston or Sudan ebolavirus) or Marburg virus); Togaviruses (e.g., Rubivirus, Alphavirus, and Arterivirus, including rubella virus); Flaviviruses (e.g., Tick- borne encephalitis (TBE) virus, Dengue (types 1, 2, 3 or 4) virus, Yellow Fever virus, Japanese encephalitis virus, yasanur Forest Virus, West Nile encephalitis virus, St. Louis encephalitis virus, Russian spring-summer encephalitis virus, and Powassan encephalitis virus); Pestiviruses (e.g., Bovine viral diarrhea (BVDV), Classical swine fever (CSFV) and Border disease (BDV)); Hepadnavirus (e.g., Hepatitis B virus, hepatitis C virus, delta hepatitis virus, hepatitis E virus, or hepatitis G virus); Rhabdo viruses (e.g., Lyssavirus, Rabies virus and Vesiculovirus (VSV)); Caliciviridae (e.g., Norwalk virus (Noro virus), and Norwalk-like Viruses, such as Hawaii Virus and Snow Mountain Virus); Coronavirus (e.g., SARS coronavirus, avian infectious bronchitis (1BV), Mouse hepatitis virus (MHV), and Porcine transmissible gastroenteritis virus (TGEV)); Retroviruses (e.g., Oncovirus, Lentivirus (e.g. HIV-1 or HIV-2) or a Spumavirus); Reoviruses (e.g., Orthoreovirus, Rotavirus, Orbivirus, and Coltivirus); Parvoviruses (e.g., Parvovirus B19); Herpesviruses (e.g., human herpesvirus, such as Herpes Simplex Viruses (HSV), e.g., HSV types 1 and 2, Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8)); Papovaviridae (e.g., Papillomaviruses and Polyomaviruses, e.g., serotypes 1, 2, 4, 5, 6, 8, 1 1, 13, 16, 18, 31, 33, 35, 39, 41 , 42, 47, 51 , 57, 58, 63 or 65, preferably from one or more of serotypes 6, 1 1 , 16 and/or 18); Adenoviruses, such as adenovirus serotype 36 (Ad-36).

Exemplary fungi include, but are not limited to, Dermatophytres, including Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton naegnini, Trichophyton mentagrophytes, Trichophyton quinckeanum, Trichophyton rubrum, Trichophyton schoenleini, Trichophyton tonsurans, Trichophyton verrucosum, T. verrucosumvar. album, var. discoides, var. ochraceum, Trichophyton violaceum, and/ or Trichophyton faviforme; Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Microsporidia, Encephalitozoon spp., Septata intestinalis and Enterocytozoon bieneusi; Brachiola spp., Microsporidium spp., Nosema spp., Pleistophora spp., Trachipleistophora spp., Vittaforma spp., Paracoccidioides brasiliensis, Pneumocystis carinii, Pythiumn insidiosum, Pityrosporum ovale, Sac aromyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp., Mucor spp., Absidia spp., Mortierella spp., Cunninghamella spp., Saksenaea spp., Alternaria spp., Curvularia spp., Helminthosporium spp., Fusarium spp., Aspergillus spp., Penicillium spp., Monolinia spp., Rhizoctonia spp., Paecilomyces spp., Pithomyces spp., and Cladosporium spp.

Exemplary parasites include, but are not limited to, Plasmodium, such as P. falciparum, P. vivax, P. malariae and P. ovale, as well as those parasites from the Caligidae family, particularly those from the Lepeophtheirus and Caligusgenera, e.g., sea lice such as Lepeophtheirus salmonis and Caligus rogercresseyi.

When referred to herein that, e.g. a "sample is from", e.g. an organism, such as a subject or pathogen, it is meant that such a sample is (or was) obtained from, e.g. an organism, such as a subject or pathogen.

A "sample organism" as used herein is an organism or part thereof, e.g. nucleic acids or proteins contained in a sample or a sample obtained from an organism or part thereof, particularly the sample organism contains nucleic acids or proteins or both.

A "sample pathogen" as used herein is a pathogen or part thereof contained in a sample or a sample from a pathogen or part thereof, particularly the sample pathogen contains nucleic acids or proteins or both.

A "sample subject" as used herein is a subject or part thereof contained in a sample or a sample from a subject or part thereof, particularly the sample pathogen contains nucleic acids or proteins or both.

A "sample" may be a biological sample or an environmental sample, e.g., surface swabs/sponges, soil, sediments, fomites, water, such as surface water, seawater, potable water, process water, waste water; food, such meat, milk, fruits, vegetables; beverages; clinical/veterinary sample, e.g., blades, cutting surfaces, catheters, needles, with a biological sample being preferred.

A biological sample preferably contains an organism or part thereof, e.g. a pathogen or part thereof of a subject or part thereof, particularly it contains proteins or nucleic acids or both, e.g. nucleic acids or proteins from an organism, such as a subject or pathogen, with nucleic acids from a pathogen being preferred or proteins from a subject being preferred. For example, a subject may contain a pathogen. If a sample is obtained from the subject, it is envisaged that said sample ideally contains a pathogen, in particular nucleic acids or proteins or both from the pathogen.

A biological sample is preferably from an organism, such as a subject or pathogen. Accordingly, the biological sample is preferably obtained from an organism, such as a subject.

A biological sample can be selected from the group consisting of whole blood, blood fractions such as plasma or serum, amniotic fluid, reproductive system secretions, synovial fluid, bone marrow, liquor, tissue or cell smear, tissue or cell swab of tissue, urine, semen, tissue, sputum, bronchial aspirate, stool, gastrointestinal secretions such as bile, lymph fluid, and lavage. A preferred biological sample is whole blood, serum or plasma. A biological sample preferably contains nucleic acids or proteins or both, e.g. nucleic acids or proteins from an organism, such as a subject or pathogen

Optionally, the biological sample can be obtained from a mucous membrane of the subject. The term "biological sample" can also include processed biological samples such as fractions or isolates, e.g., nucleic acids, proteins or isolated cells. Preferably, the biological sample contains nucleic acids, e.g., genomic DNA or mRNA of, e.g. a pathogen, such that the sequence of the nucleic acids can be determined.

Preferably, the biological sample in addition to nucleic acids or alternative to nucleic acids contains proteins, of, e.g. a subject or pathogen, such that the sequence, i.e., amino acid sequence of proteins can be determined.

In an embodiment, the biological sample can be one that is obtained from a tissue showing signs of a disease state, e.g., showing signs of infection or signs of cancer. The sample is analyzed according to the methods of the invention and during the method or thereafter is not normally returned to the body. In most embodiments, the presence of the subject's body is not necessary in order to carry out the methods of the invention.

The biological sample, e.g., blood plasma, may contain free circulating nucleic acids, which may preferably comprise nucleic acids of the subject and nucleic acids being not of the subject, e.g., those of a pathogen. In one embodiment the biological sample can be diluted or concentrated. In another embodiment the sample is processed prior to sequencing, preferably the sample is purified to remove cellular components, such as lipids and proteins, prior to sequencing. In one embodiment, the biological sample is processed prior to sequencing such that only cell-free nucleic acids are sequenced. The biological sample may, however, be processed prior to sequencing such that cells are lysed to release their nucleic acids.

The biological sample, e.g., blood plasma, may in addition to free circulating nucleic acids or alternatively to free circulating nucleic acids contain proteins from a subject or a pathogern or both.

In another embodiment the sample is processed prior to sequencing, preferably the sample is purified to remove cellular components, such as lipids and nucleic acids, prior to sequencing. In one embodiment, the biological sample is processed prior to sequencing such that only proteins are sequenced. The biological sample may, however, be processed prior to sequencing such that cells are lysed to release their proteins.

Tissues (comprising cells) of the subject, e.g. a patient from which the biological sample can be obtained include, but are not limited to, throat, mouth, nasal, heart, brain, bone marrow, breast, stomach, gall bladder, duodenum, intestinal, colon, skin, joint, liver, kidney, pancreatic, lung, neuronal cervical, vaginal, uteral, urethral, rectal, penial, and muscle. Tissues of the subject may also include cancerous tissue (comprising cancerous cells), e.g. cancerus tissue of any of the aforementioned organs or structures. Any suitable method for obtaining the biological sample from the subject, e.g. patient and/or from an appropriate tissue can be used in connection with the present invention.

"Biological sequence data" or "sequence data" is the information obtained by way of sequencing genetic material of an organism, such as a pathogen. As is commonly known, the information is a sequence of the nucleotides G, A, C, T/U.

Sequencing of genetic material may be carried out using ultradeep or high-throughput sequencing methods. In preferred embodiments of the invention, the sequencing is performed by molecular high-throughput sequence analysis, i.e., by nextgeneration sequencing (NGS) or third generation sequencing, such as by the Illumina/Solexa or the Oxford Nanopore methodology.

"Biological sequence data" or "sequence data" also encompasses information obtained by way of sequencing proteins of an organism, such as a subject or pathogen; or information obtained by way of (in silico or in vitro) translating the information obtained by way of sequencing genetic material of an organsim. Indeed, as is commonly known, a triplett of nucleotides encodes an amino acid. Bearing the latter in mind, it is apparent that the term "biological sequence data" or "sequence data" also encompasses amino acid sequences of proteins.

As is further commonly known, the information of a sequenced protein is a sequence of amino acids, such as alanine, arginine, asparagine, aspartic acid, cysteine, glutamic acid, glutamine, glycine, histidine, isoleucine, leucine, lysine, methionine, phenylalanine, proline, serine, threonine, tryptophane, tyrosine, valine, selenocysteine, or pyrrolysine.

Of note, amino acids may be natural amino acid or non-natural amino acids, such as D-amino acids, homo amino acids, N-methyl amino acids, alpha-methyl amino acids, beta (homo) amino acids, gamma amino acids. Non-natural amino acids may occur in nature, e.g. hydroxyproline (Hyp), beta-alanine, citrulline (Cit), ornithine (Orn), norleucine (Nle), 3-nitrotyrosine, nitroarginine, pyroglutamic acid, hydroxyproline (Hyp), nachtylalanine (Nal), Abu, DAB, methionine sulfoxide, methionine sulfone, or may be chemically synthetisized.

When used herein, for avoidance of doubt, "sequenced protein" means a protein which is sequenced de novo to derive its amino acid sequence or which has already been sequenced or the (amino acid) sequence of which is derived from translating the nucleotide sequence encoding said protein.

Protein sequencing may be achieved by the so-called Edman analysis, also called Edman degradation or, e.g. by sequencing a protein, ideally in its denatured unfolded state with a nanopore or subnanopore; or by sequencing a folded protein with a nanopore; see, e.g. Timp and Timp, Sci. Adv. 6: eaax8978 (2020).

In the methods of the present invention it is preferred that biological sequence data of the pathogen comprise a sequence of deoxyribonucleic acid, DNA, called genome, or a sequence of ribonucleic acid, RNA, called transcriptome, of said pathogen

The term "genome" relates to the total amount or parts thereof of information of the genetic material of an organism, such as a subject or pathogen.

In the methods of the present invention, it is preferred that a genome can be assembled from a plurality of short sequences, called reads, of different portions of the genome, particularly of the genome of the pathogen.

A "contig" or "contigs" when referred to herein is set of fragments of a nucleic acid, e.g. of a genome of an organism, such as a subject or pathogen, and they together represent the "whole", e.g. genome or parts thereof or transcriptome or parts thereof, e.g. of said organism, e.g. subject or pathogen. Said term encompasses also expressed sequence tags (ESTs). In the context of the present invention, contig(s) are sequenced e.g. in the form of a sequence read. Ideally the sequence reads overlap so as to allow an assembly and thus a reconstruction of the "whole". Due to the overlap, contigs can be assembled to reconstruct the genome or parts thereof or the transcriptome or parts thereof, e.g. of an organism, such as a pathogen or subject. Contigs may thus refer both to overlapping nucleotide sequences (inferred from nucleic acid fragments) or to overlapping physical segments (i.e., fragments) of a genome or parts thereof or of a transcriptome or parts thereof, e.g. of an organism, such as a pathogen or subject, depending on the context.

As said, contigs can be assembled, i.e., a genome or parts thereof or a transcriptome or parts thereof can be reconstructed. This is possible, if contigs ideally overlap. "Sequence assembly" or "assembly" as also used herein, refers to aligning and merging nucleic acid fragments or their nucleotide sequence, respectively, to reconstruct the original (full) sequence. This is needed as sequencing technology cannot read whole genomes or larger parts thereof in one go.

Sequencing nucleic acids to infer their nucleotide sequence and, if needed, assembling them is done in the context of obtaining or achieving biological sequence data which is then processed by the means and methods of the present invention described herein.

A "read" or "sequence read" is an inferred sequence of bases (or base probabilities) corresponding to all or part of a nucleic acid fragment which is or was sequenced. For the purpose of the present invention, reads of a length of 20-1500 base pairs (bp) are preferably referred to as short. However, longer reads, i.e. reads longer than 1500 bp are also possible and are encompassed by the present invention when saying "read".

A read of e.g., a contig may be assigned to an organism, such as a pathogen or subject, preferably mapped to the genome of the respective organism, such as a pathogen or subject. In a preferred embodiment, the reads are classified to a specific organism, such as a pathogen or subject. In another preferred embodiment, reads can be normalized by their abundance.

The term "genetic material" includes isolated nucleic acid, either DNA or RNA, a section of a double helix, a section of a chromosome, or an organism's , such as a subject's or pathogen's entire genome or an organism's cells, such as a subject's or pathogen cells.

According to the invention, "nucleic acid" is preferably deoxyribonucleic acid (DNA) or ribonucleic acid (RNA). Nucleic acids include genomic DNA, cDNA, mRNA, recombinantly produced and chemically synthesized molecules. A nucleic acid may be present as a single-stranded or double-stranded and linear or covalently circularly closed molecule, as well as mixtures thereof. A nucleic acid can be isolated. Preferably, the nucleic acid is a free circulating DNA and/or RNA molecule. In one embodiment, the term "nucleic acid" is also understood to mean "nucleic acid sequence". Further, prior to sequencing, the nucleic acids can be processed, for example, enriched or amplified. In cases where the nucleic acid obtained from the sample is RNA, the RNA can be reverse transcribed into DNA for sequencing or the RNA itself can be sequenced.

The term "transcriptome" relates to the set of all RNA molecules, including mRNA, rRNA, tRNA, and other non-coding RNA produced in one cell or a population of cells of an organism, such as a pathogen or subject. In context of the present invention the transcriptome means the set of all RNA molecules produced in one cell of an organism, such as a pathogen or subject, a population of cells of an organism, such as a pathogen or subject, or all cells of an organism, such as a pathogen or subject.

In the methods of the present invention, it is also or alternatively preferred that biological sequence data of the subject or pathogen comprise a sequence of amino acids, i.e., (an) amino acid sequence(s).

The term "protein" encompasses both peptides and polypeptides. The term "protein" also refer to naturally modified proteins, wherein the modification is effected e.g. by post-translational modifications like glycosylation, acetylation, phosphorylation and the like. Such modifications are well known in the art. Similarly, the term "protein" also refers to non-naturally modified proteins, e.g. proteins containing one or more non-natural amino acids, e.g. as described herein.

A protein is composed of two or more amino acids, whereby the amino acids are bound among one another via a peptide bond. Such a chain of amino acids is the amino acid sequence of a protein.

A "polypeptide" as used herein describes a group of molecules, which contains 30 or more amino acids, such as 40, 50, 60, 70, 80, 100, 200, 300, 400, 500, 600, 700, 800, 900, 1000 amino acids or more. Also in line with the definition, the term "polypeptide" describes fragments of proteins as long as these fragments contain 30 or more amino acids, such as 40, 50, 60, 70, 80, 100, 200, 300, 400, 500, 600, 700, 800, 900, 1000 amino acids or more.

Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc.

A "peptide" as used herein describes a group of molecules, which contains less than 30 amino acids, such as 25, 20, 15, 10, 9, 8, 7, 6 or 5 amino acids or less, e.g., 4, 3, 2 amino acids. Also in line with the definition, the term "peptide" describes fragments of proteins as long as these fragments contain less than 30 amino acids, such as 25, 20, 15, 10, 9, 8, 7, 6 or 5 amino acids or less, e.g., 4, 3, 2 amino acids.

The term "proteome" when used in the context of the present invention refers to the entire set of proteins that is, or can be, expressed by a genome, comprised by a cell, tissue, or organism, e.g. at a certain time. Said term also encompasses the set of expressed proteins in a given type of cell or organism, at a given time, under defined conditions.

In the methods of the present invention it is preferred that biological sequence data of the pathogen comprise a sequence of deoxyribonucleic acid, DNA, called genome, or a sequence of ribonucleic acid, RNA, called transcriptome, or a sequence of two or more amino acids, called protein, or sequences of one or more proteins, called proteome, of said organism, such as a subject or pathogen.

In the methods of the present invention, it is preferred that a proteome can be assembled from a plurality of proteins, e.g. peptides or polypeptides of, e.g. different portions of a protein, particularly of the proteome of the subject or pathogen. Also a protein can be assembled from a plurality of peptides or polypeptides which may ideally overlap.

When used herein, the term "anti-infective" encompasses any medicine that is capable of inhibiting the spread of a pathogen or killing the pathogen. This term encompasses antibiotics, antifungals, antihelmintics, antimalarials, antiprotozoals, antituberculosis agents, and antivirals, with antibiotics being preferred.

"Sensitivity" to an anti-infective when used herein means that a pathogen is susceptible to an anti-infective. That is, an anti-infective is capable of inhibiting the spread of a pathogen, growth of a pathogen or killing the pathogen. Put differently, "sensitivity" is the absence of resistance.

"Resistance" to an anti-infective when used herein means that a pathogen is not susceptible to an anti-infective. That is, an anti-infective is not capable of inhibiting the spread of a pathogen, growth of a pathogen or killing the pathogen.

For coming up with the means and methods of the present invention, the present inventors used genomes of resistancedescribed pathogens to train a neural network, e.g. CNN (convolutional neural network), LSTM etc.; see, e.g. Goodfellow, I., Bengio, Y. & Courville, A. Deep Learning (MIT Press, 2016).

Thanks to the means and methods of the present invention, only the sequence/genome/RNA of the pathogen is required to discover resistance phenotypes or, vice versa sensitivity phenotypes. The known marker sequences for the resistances are not required.

However, in addition, although it is not required in the context of the present invention, these genomes may be described/characterized in terms of sensitivity or resistance to anti-infectives using, e.g. phenotyping methods.

In the methods of the present invention, the neural network is preferably a convolutional neural network (CNN) a long short-term memory (LSTM), a recurrent neural network or a transformer type neural network. A transformer type neural network is, e.g. described by Vaswani et al., 31st Conference on Neural Information Processing Systems (NIPS 2017): http://papers.nips.cc/paper/7181-attention-is-all-you-need.pdf.

A preferred neural network architecture consists of an input tensor (size 4 x 5,700,000). The next layer is preferably a 2D convolution layer with a kernel size of 200 x 4 and a total of 128 kernels (also known as channel), followed by a 2D max pooling layer of size 200 x 1. These two layers are then preferably followed by two blocks of 2D convolution and 2D max pooling layers with the same parameters as described above, but with 256 and 512 kernels. Then, the output of the last 2D max pooling layer is preferably flattened to a 1D tensor and fed to a fully connected layer with 1,024 neurons, preferably followed by a softmax layer to yield a discrete output vector for the different potential resistances, e.g. 17 different potential resistances.

The identical preferred neural network architecture can be used for classifying sensitivities as described herein.

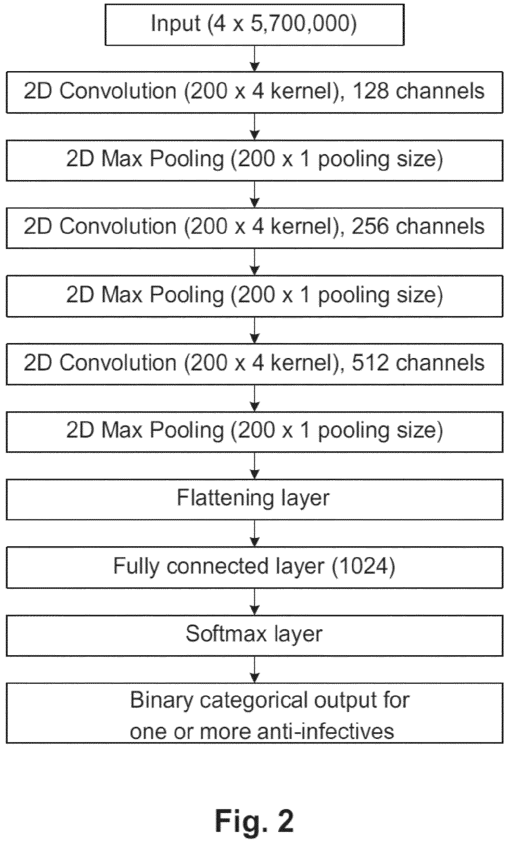

Figure 2 illustrates a preferred neural network architecture.

Preferably, for training, augmented genomes, e.g. those augmented as described herein, and the corresponding vectors of known (or, sometimes referred to herein as "measured vector") resistances or sensitivities, respectively are taken and the augmented genomes are fed to the neural network as input and the output vector is compared to the vector of known (or, sometimes referred to herein as "measured vector") resistances or sensitivities, respectively. Then, preferably, an error using a loss function is calculated and the error is used, in combination with automated differentiation to adjust the weights and biases (offsets) of the neural network. A process known as backpropagation, e.g. as described in Rumelhart et al., Nature 323, 533-536 (1986): http://www.nature.com/nature/journal/v323/n6088/abs/323533a0.htm l.

Backpropagation is preferably performed for as many rounds (epochs) of training as required to achieve a certain desired accuracy. In order to prevent early overfitting, a process called dropout is preferably used, where a certain percentage of weights for each epoch of training is randomly deleted. As an optimizer, the Adam optimizer and a learning rate (the extent of gradient descent) is preferably used, here, e.g. 0.00001 is used. An Adam optimizer is, e.g. described by Kingma and Ba in http://arxiv.org/abs/1412.6980.

Figure 2 illustrates a preferred neural network training.

In the methods of the present invention, it is preferred that the neural network is trained on a plurality of specimen sequences with an Adam optimizer, and a learning rate of 0.00001 for preferably 146 epochs

In the methods of the present invention, it is preferred that sequence data input to the neural network can either be the nucleotide sequence or any other summary statistic (e.g. k-mers) or preprocessed from of the original sequence.

Preferably, in the methods of the present invention, sequence data input to the neural network comprises elements A, C, G, T of DNA or RNA encoded as one-hot representations 0001, 0010, 0100, and 1000.

For training of the neural network, preferably a CNN, convolution operations moving along the genomic sequence of each genome or transcriptome in the database may be used, together with weight updates through backpropagation, to learn the specific features of anti-infective resistant and anti-infective sensitive genomes/transcriptomes.

Preferably, in the methods of the present invention, the neural network has one or more convolution layers and at least one fully connected layer. The neural network can consist of one to x layers with several dozens of channels/kernels per layer.

The channels can be one-dimensional or two-dimensional, depending on the genomic features of the input.

Preferably, in the methods of the present invention, each respective convolution layer has 128, 256, 512 or more kernels per layer with a shape of 200 by 4 per kernel

Preferably, in the methods of the present invention, each respective convolution layer applies dropout of 10%, 15%, 20%, 21%, 22%, 23%, 24%, 25%, 30% for training to avoid overfitting by randomly setting weights to zero, with 20% being preferred.

The output provided by the neural network, preferably a CNN, can either be a binary classification of a range of anti-infectives to indicate presence or absence of resistance for either one of those or even only a classification for the presence or absence of resistance against a single anti-infective.

The error or loss functions used for training can either be a cross entropy loss function, binary cross entropy loss function or any derived loss function to penalize certain features/outcomes during training.

Particularly preferred hyperparameters and training applied in the methods of the present invention are a learning rate of 0.00001, an Adam optimizer, a loss function of the binary cross entropy type and a dropout of 20% for every convolution layer at training; all of which are described herein.

For inference of anti-infective resistance, the same preprocessing may be used as for training and the output of the forward propagation through the neural network, preferably a CNN, may be used as the vector of predictions, which then may be subjected to a softmax function or any other function to generate predictions for each anti-infective the neural network, preferably a CNN, has been trained to classify.

As soon as this database is available, and a corresponding training of the neural network, preferably a CNN, has taken place, any sequence data (or k-mer frequencies or similar characteristics) can be entered into the neural network, preferably a CNN, and a corresponding classification can take place.

Preferably, in the methods of the present invention for classifying resistances of a sample pathogen against one or more anti-infectives, resistances of a pathogen against the one or more anti-infectives are signified either by binary values from {0, 1} or by rational values from [0, 1], wherein 0 signifies no anti-infective resistance.

Preferably, in the methods of the present invention for classifying sensitivities of a sample pathogen against the one or more anti-infectives are signified either by binary values from {0, 1} or by rational values from [0, 1], wherein 0 signifies anti-infective tolerance.

Preferably, in the methods of the present invention, the neural network uses a cross entropy loss function for training, wherein the neural network has one or more max pooling layers, and a softmax classification layer to generate classification predictions.

In particular, in the context of the methods of the present invention data augmentation is performed. Data augmentation is performed with the aim of maximizing diversity of input data to the neural network in order to provide a maximum of diverse input genomes and process them such that they can be used as an input to the neural network. For this, a genome is partitioned in multiple parts, e.g. in parts s₁ to sₙ, each part not exceeding a certain length.

An exemplary certain length in case of nucleic acids is 1 kbp, 5 kbp, 10 kbp, 20 kbp, 30 kbp, 40 kbp, 50 kbp, 60 kbp, 70 kbp, 80 kbp, 90 kbp or 100 kbp, with 20 kbp being preferred.

Then, out of these parts, an augmented genome is assembled by randomly sampling from the set of parts and concatenating those parts until a predetermined maximum total length of all concatenated parts is reached which then constitutes the augmented genomes. An exemplary predetermined maximum total length in case of nucleic acids is 1000 kbp, 2000 kbp, 3000 kbp, 4000 kbp 5000 kbp, 5700 kbp, 6000 kbp, 7000 kbp, 8000 kbp, 9000 kbp or 10000 kbp, with 5700 kbp being preferred.

For this, an amino acid sequence of a protein or amino acid sequences of proteins which may constitue a proteome is/are partitioned in multiple parts, e.g. in parts s₁ to sₙ, each part not exceeding a certain length.

An exemplary certain length in case of an amino acid sequence of a protein is 10, 20, 30, 40, 50, 60, 70, 80, 90, 100, 120, 140, 160, 180, 200, 220, 240, 260, 280, 300, 320, 340, 360, 380, 400, 420, 440, 460, 480, 500, 1000, 2000, 3000, 4000, 5000 or more amino acids, of course, dependent on the length of the amino acid sequence.

Then, out of these parts, (an) augmented amino acid sequence(s) is/are assembled by randomly sampling from the set of parts and concatenating those parts until a predetermined maximum total length of all concatenated parts is reached which then constitutes the augmented amino acid sequence(s).

An exemplary predetermined maximum total length in case of amino acids is 100, 150, 200, 250, 300, 350, 400, 450, 500, 550, 600, 650, 700, 750, 800, 850, 900, 950, 1000, 1500, 2000, 2500, 3000, 3500, 4000, 4500, 5000, 10000, 20000, 30000, 40000, 50000, 60000, 70000, 80000, 90000, 100000, 200000, 300000, 400000, 500000, 1000000 or more amino acids.

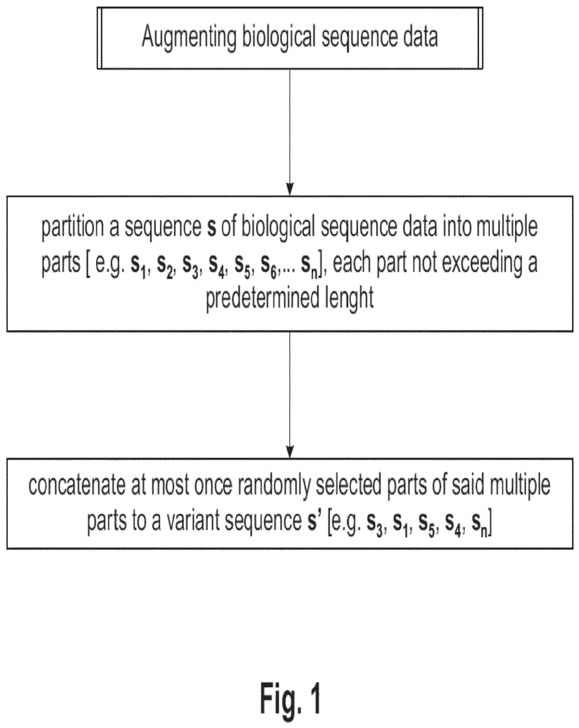

See also Figure 1 which illustrates a preferred data augmentation as applied in the methods of the present invention.

Hence, as referred to in the methods of the present invention, it is the augmentation of sequence data, particularly the augmentation of sequence data of a first plurality of biological sequences obtained from a sample organism, such as a sample pathogen to train a neural network, NN, preferably the organism, e.g. a subject or pathogen having a known property towards an external stimulus, e.g. resistances (or sensitivities) against one or more anti-infectives, to sequence data of a second plurality of variant biological sequences by generating sequence data of multiple variants of each sequence of the first plurality of sequences, preferably each variant carrying the same property towards the external stimulus, e.g. resistances (or sensitivities) against one or more anti-infectives as the corresponding sequence of the first plurality of sequences, wherein
generating sequence data of multiple variants for each sequence of the first plurality of sequences, referred to as first sequence comprises:
partitioning the first sequence into multiple parts, each part not exceeding a predetermined length; and
generating multiple variants of the first sequence by concatenating at most once randomly selected parts of said multiple parts to respective variants of the first sequence, thereby obtaining sequence data of the second plurality of biological sequences.

That said, the present invention also relates to an independent (in vitro) method for augmenting sequence data of a first plurality of biological sequences from a sample organism to train a neural network, NN, preferably the organism having a known property towards an external stimulus, to sequence data of a second plurality of variant biological sequences by generating sequence data of multiple variants of each sequence of the first plurality of sequences, preferably each variant carrying the same property towards the external stimulus as the corresponding sequence of the first plurality of sequences, wherein
generating sequence data of multiple variants for each sequence of the first plurality of sequences, referred to as first sequence comprises:
partitioning the first sequence into multiple parts, each part not exceeding a predetermined length; and
generating multiple variants of the first sequence by concatenating at most once randomly selected parts of said multiple parts to respective variants of the first sequence, thereby obtaining sequence data of the second plurality of biological sequences.

As described herein, the sequence data may preferably be a sequence of nucleotides (nucleotide sequence) or a sequence of amino acids (amino acid sequence).

The sequence of nucleotides may comprise G, C, T and/or A/U. These nucleotides may also be derivatives or (chemical) modifications of G, C, T or A/U.

The sequence of amino acids may comprise alanine, arginine, asparagine, aspartic acid, cysteine, glutamic acid, glutamine, glycine, histidine, isoleucine, leucine, lysine, methionine, phenylalanine, proline, serine, threonine, tryptophane, tyrosine, valine, selenocysteine, or pyrrolysine. These amino acids may also be derivatives, e.g. formly-methionine or (chemical) modifications of the afore-mentioned amino acids.

Without being bound by theory, in case of augmenting the amino acid sequence of a protein, it is assumed that such an augmented amino acid sequence can be useful for training a NN so that the NN can, e.g. infer from the augmented amino acid sequence one or more potential folds or structures which the protein may adopt.

Preferably, in the independent method of the present invention, the sample organism is a sample subject or sample pathogen as described herein. As described herein, a "sample organism" is an organism or part thereof, e.g. nucleic acids or proteins contained in a sample or a sample obtained from an organism or part thereof, particularly the sample organism contains nucleic acids or proteins or both. A sample subject or sample pathogen contains preferably nucleic acids or proteins or both. In a preferred embodiment of the independent method of the present invention, the sample organism contains proteins. The same applies to a sample subject or sample pathogen.

Preferably, in said independent method the known property of an organism towards an external stimulus is, e.g. a resistance against a medicament, e.g., resistance against one or more anti-infectives; sensitivity against a medicament, e.g. sensitivity against one or more anti-infectives.

The term "property" also includes a trait.

Since the term "organism" also encompasses a part of an organism, e.g. proteins as described herein, a known property of a protein towards an exernal stimulus may be the function of said protein. An external stimulus with respect to the function of said protein may be a signal, e.g. the activation, inhibition, translocation, etc. of said protein.

In the independent method of the present invention, it is preferred that passing the biological sequence data to the input layer of the NN further comprises:
generating augmented biological sequence data from the obtained biological sequence data by partitioning the obtained biological sequence data into multiple parts, each part not exceeding a predetermined length;
concatenating at most once randomly selected parts of said multiple parts to a variant of the obtained biological sequence data; and
passing the augmented biological sequence data in lieu of the obtained biological sequence data to the input layer of the NN.

Also, in the independent method of the present invention, it is preferred that generating sequence data of multiple variants of each sequence of the first plurality of sequences comprises generating at least 10 variants, at least 15 variants, at least 20 variants, at least 25 variants, at least 30 variants, at least 35 variants, at least 40 variants, at least 45 variants, at least 50 variants, or more.

All embodiments, preferred embodiments, etc. as described herein also pertain to the independent method of the present invention, mutatis mutandis.

For example, preferably, in the independent method of the present invention, biological sequence data of the organism comprise a sequence of deoxyribonucleic acid, DNA, called genome, or a sequence of ribonucleic acid, RNA, called transcriptome, or a sequence of two or more amino acids, called protein, or sequences of one or more proteins, called proteome, of said organism, such as a subject or pathogen.

Preferably, in the independent method of the present invention a genome can be assembled from a plurality of short sequences, called reads, of different portions of the genome.

Preferably, in the independent method of the present invention, sequence data input to the neural network comprise elements A, C, G, T of DNA or RNA encoded as one-hot representations 0001, 0010, 0100, and 1000.

In the methods of the present invention, it is preferred that a proteome can be assembled from a plurality of proteins, e.g. peptides or polypeptides of, e.g. different portions of a protein, particularly of the proteome of the subject or pathogen.

Preferably, in the independent method of the present invention, the neural network, NN, comprises one of: a convolutional neural network, CNN, a long short-term memory, LSTM, a recurrent neural network and a transformer type neural network.

Preferably, in the independent method of the present invention, the neural network has one or more convolution layers and at least one fully connected layer.

Preferably, in the independent method of the present invention, the each respective convolution layer has 128, 256, 512 or more kernels per layer with a shape of 200 by 4 per kernel.

Preferably, in the independent method of the present invention, wherein each respective convolution layer applies dropout of 20% for training to avoid overfitting by randomly setting weights to zero.

Preferably, in the independent method of the present invention, the NN uses a cross entropy loss function for training, wherein the neural network, NN, has one or more max pooling layers, and a softmax classification layer to generate classification predictions.

Preferably, in the independent method of the present invention, the neural network is trained on a plurality of specimen sequences with an Adam optimizer, and a learning rate of 0.00001 for preferably 146 epochs.

The present invention further relates to a computer program product adapted to carry out the method of the present invention.

The computer program product may use hardware, firmware, software or a combination thereof to perform the various steps and operations described herein.

A computing system suitable for performing the activities described herein may include a server. Such a server may include a central processor (CPU) coupled to a random-access memory (RAM) and to a read-only memory (ROM). ROM may also be other types of storage media to store programs, such as programmable ROM, erasable PROM, etc. Processor may communicate with other internal and external components through input/output (I/O) circuitry and bussing to provide control signals and the like. Processor carries out a variety of functions as are known in the art, as dictated by software and/or firmware instructions.

Server may also include one or more data storage devices, including hard drives, CD-ROM drives and other hardware capable of reading and/or storing information, such as DVD, etc. In one embodiment, software for carrying out the herein described steps may be stored and distributed on a CD-ROM or DVD, a USB storage device or other form of media capable of portably storing information. These storage media may be inserted into, and read by, devices such as CD-ROM drive, disk drive, etc. Server may be coupled to a display, which may be any type of known display or presentation screen, such as LCD, plasma display, cathode ray tube (CRT), etc. A user input interface is provided, including one or more user interface mechanisms such as a mouse, keyboard, microphone, touchpad, touch screen, voice-recognition system, etc.

Server may be coupled to other devices, such as sources, detectors, etc. The server may be part of a larger network configuration as in a global area network (GAN) such as the Internet, which allows ultimate connection to various landline and/or mobile computing devices.

### Description of the Figures:

### Figure 1: Data augmentation

A fixed size input and maximizing diversity of training data for the neural network is preferably generated. Data augmentation is preferably performed to maximize diversity of input data to the neural network in order to provide a maximum of diverse input genomes, based on a limited number of real-world sequenced genomes and process them such that, e.g. all of these augmented genomes have the same size and thus can be used as an input to the neural network. For this, a sequence "s" of biological sequence data is partitioned into multiple parts (e.g. s₁, s₂, s₃, s₄, s₅, s₆, ...sₙ], each part not exceeding a predetermined length. The starting point in the sequence "s" for the partitioning may be chosen randomly. Then, parts of said multiple parts are at most once randomly selected and concatenated to a variant sequence "s'" (e.g. s₃, s₁, s₅, s₄, sₙ).

For the sake of discussion, it is assumed that part s₂ comprises a known marker sequence for a resistance or a sensitivity to an anti-infective. Furthermore, it is assumed that s₂ is absent in the exemplary variant sequence s' (s₃, s₁, s₅, s₄, sₙ). Due to potentially undescribed effects/dependencies/correlations within for example parts s₃ and s₅, which may indirectly cause the resistance or sensitivity to the anti-infective, the variant sequence s' comprises a sequence data pattern linked to the resistance or sensitivity. Thus even with absent marker sequences each variant carries the same resistances against the one or more anti-infectives as the corresponding sequence of the first plurality of sequences.

By using a plurality of variants being created as mentioned above, to train the neural network, the neural network is trained to recognize the potentially undescribed effects/dependencies/correlations which may indirectly cause the resistances or sensitivities to the anti-infectives.

The trained neural network may then predict resistances and/or sensitivities based on incomplete genoms being present in native samples of the subject. Thus, after the sequenced samples are converted into machine readable data, the neural network may provide information about resistances and/or sensitivities after a period being shorter than prior art methods which look for marker sequences in the samples.

### Figure 2: A preferred neural network architecture

A preferred neural network architecture consists of an input tensor (size 4 x 5,700,000), e.g. 4 nucleotides G, A, C, T and a sequence length of 5.7 million base pairs. The next layer is a 2D convolution layer with a kernel size of 200 x 4 and a total of 128 kernels (also known as channel), followed by a 2D max pooling layer of size 200 x 1. These two layers are then followed by two blocks of 2D convolution and 2D max pooling layers with the same parameters as described above, but with 256 and 512 kernels. Then, the output of the last 2D max pooling layer is flattened to a 1D tensor and fed to a fully connected layer with 1,024 neurons, followed by a softmax layer to yield a discrete output vector for the 17 different potential resistances.

The output vector is compared to the vector of measured resistances (or sensitivities, respectively). Then an error using a loss function is calculated and the error is used, in combination with automated differentiation to adjust the weights and biases (offsets) of the neural network. A process known as backpropagation. This is performed for as many rounds (epochs) of training as required to achieve a certain desired accuracy. In order to prevent early overfitting, a process called dropout is used, where a certain percentage of weights for each epoch of training is randomly deleted. As an optimizer, the Adam optimizer and a learning rate (the extent of gradient descent) is used, here, e.g. 0.00001 is used.

### Figure 3: Neural network training

For a preferred training, augmented genomes and the corresponding vectors of known resistances (or sensitivities, respectively) to the antibiotics "AB", e.g AB₁, AB₂, AB₃, ABₙ as anti-infectives are taken and the augmented genomes are fed to the neural network as input. Note that the genomes "G", e.g. G₁, G₂, G₃, G₄, Gₘ may have different lengths or the same length.

By providing both, augmented for resistances and sensitivities, the neural network may be trained to provide classification predictions for the optimal anti-infective.

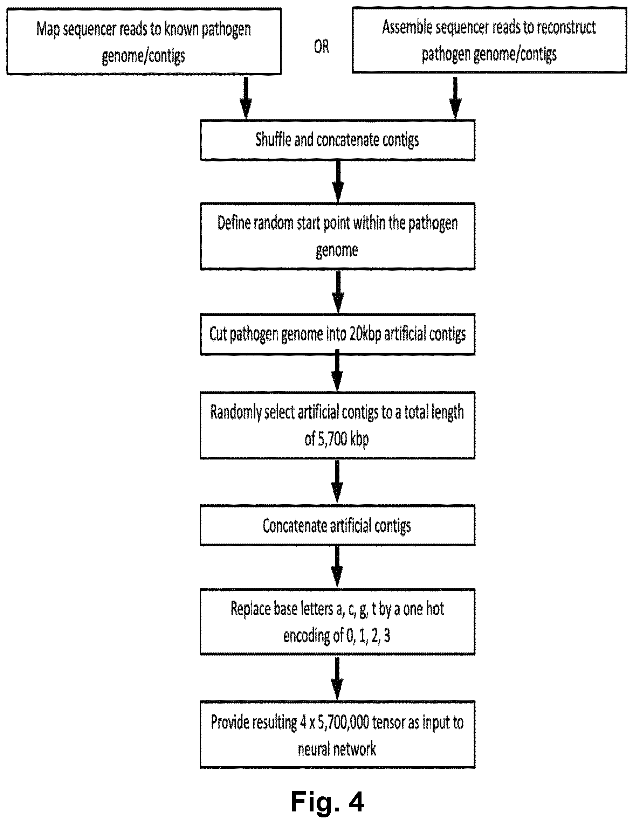

### Figure 4: Data preprocessing strategy

For training of a neural network according to the invention, all relevant parts of a K. pneumoniae genome, including all genomic and plasmid resistance factors may be shown to the neural network.

As individual genomes from the same genus are typically of different lengths and neural networks require a somewhat fixed length input, a preprocessing strategy was devised to come up with one preprocessed genome length for all genomes as shown in figure 4. First, all contigs of a genome are taken and randomly shuffled. Subsequently, the contigs are concatenated to a virtual genome.

Then, a random starting point within the virtual genome is chosen to cut the genome into 20 kbp fragments in this example. From the set of 20 kbp fragments, sampling with replacement 285 times is performed to achieve a total sequence length of 5,7 Mbp from each virtual genome. This results in a fixed input size of 5,7 Mbp for any K. pneumoniae genome sequenced, irrespective of the in vivo genome size, plasmid content or assembly quality, i. e. the number of contigs produced by the sequencing pipeline.

For any K. pneumoniae genome not of 5.7 Mbp in vivo genome size, this means either slight oversampling, for about 90% of genomes, or undersampling, for about 10% of genomes. Then those 285 fragments of 20 kbp length are concatenated. Furthermore, the individual nucleotide characters to integers in a one hot encoding fashion. The resulting tensor of shape 4 x 5,700,000 is then used as the actual input to the CNN. The starting data of that approach could either be assembled reads from short read sequencing and subsequent assembly or long read sequencing, e.g. Oxford nanopore, which could be directly treated as contigs.

The training of the CNN until convergence may for example be defined by no apparent overfitting or improvement of accuracy for 5 epochs. Typically, it takes 120 to 150 epochs of training until convergence.

As an optimizer, for example the Adam optimiser for gradient descent may be used with a learning rate of 1x10⁻⁵. For initialization of weights and biases, for example Normal initialization according to He may be used. To counter overfitting, dropout with a probability of 0.2 may be used at training.

Furthermore, for example the program Tensorflow 1.15 may be used as a framework for training and testing of the neural networks. All training may be run using Python 3.6.7 on OpenSuSE Linux 15.0. As GPUs for training, Nvidia Tesla V100s with 32Gb of memory, with the Nvidia libraries CUDA 10.1 and CuDNN 7.6.5 may be used.

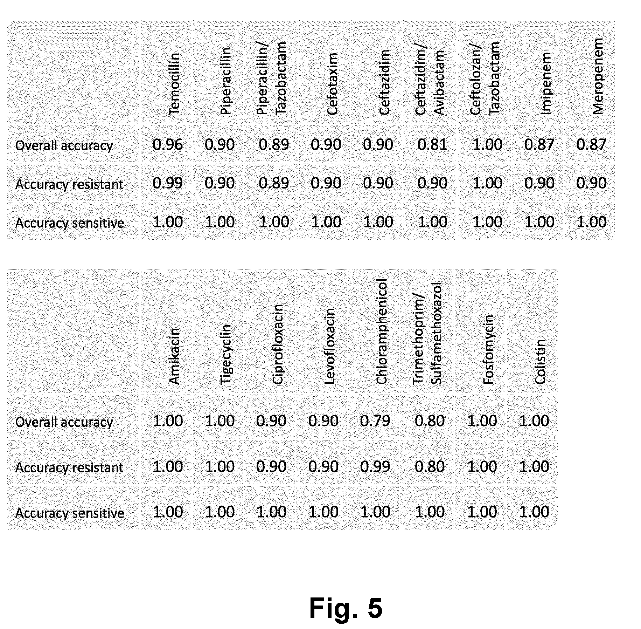

### Figure 5: Overall, resistance specific and sensitivity specific accuracies of predictions of the CNN for 17 different antimicrobials

Figure 5 shows that a CNN neural network can be used to determine antimicrobial resistance information only from the K. pneumoniae genome sequence. The CNN architecture, which expects genome fragments of 20kbp as inputs, can determine with high accuracy whether a bacterial sample is sensitive to a range of antimicrobials as shown in the tables of figure 5. The CNN can also determine whether a K. pneumoniae genome holds resistance against one or several of 17 microbials, with a slightly lower accuracy of 79% for chloramphenicol and up to 100% of cases for the antimicrobial drug combination Ceftolozan/Tazobactam. The sample size was 100. Thus, data augmentation according to the present invention improved the training of the CNN neural network. To further improve the CNN neural network such that the CNN can also analyze other bacterial species, a bigger sample size may be chosen. Furthermore, the K. pneumoniae genomes came from a similar source. By using genomes from different sources, the training data may be improved further.

The present invention may also be characterized by the following items:
(1) A computer-implemented method for classifying resistances of a sample pathogen against one or more anti-infectives from biological sequence data of said sample pathogen, the method comprising:
   augmenting sequence data of a first plurality of biological sequences of pathogens, different from the sample pathogen, having known resistances against the one or more anti-infectives, to sequence data of a second plurality of variant biological sequences by generating sequence data of multiple variants of each sequence of the first plurality of sequences, each variant carrying the same resistances against the one or more anti-infectives as the corresponding sequence of the first plurality of sequences, wherein
   generating sequence data of multiple variants for each sequence of the first plurality of sequences, referred to as first sequence comprises:
      partitioning the first sequence into multiple parts, each part not exceeding predetermined length; and
      generating multiple variants of the first sequence by concatenating at most once randomly selected parts of said multiple parts to respective variants of the first sequence, thereby obtaining sequence data of the second plurality of biological sequences;
      training a neural network, NN, via a input layer, specimen sequence data of the second plurality of biological sequences of pathogens labelled with known resistances of the respective pathogens, using forward propagation that repeatedly compares outputs of the neural network with corresponding ground truth labels to calculate an error using a loss function, and using backpropagation to calculate a gradient of the loss function to adjust the weighting values of the NN,
      obtaining, after training the NN, biological sequence data for inferring resistances of a sample pathogen; and
      passing said biological sequence data to an input layer of the NN with weights and biases after teaching incorporated;
      generating, via an inference output layer of the NN, classification predictions of resistances of the sample pathogen against the one or more anti-infectives obtained from output of the neural network.
(2) The method of the preceding item, wherein passing said biological sequence data to the input layer of the NN further comprises:
   generating augmented biological sequence data from the obtained biological sequence data by partitioning the obtained biological sequence data into multiple parts, each part not exceeding a predetermined length;
   concatenating at most once randomly selected parts of said multiple parts to a variant of the obtained biological sequence data; and
   passing the augmented biological sequence data in lieu of the obtained biological sequence data to the input layer of the NN.
(3) The method of any of the preceding items, wherein generating sequence data of multiple variants of each sequence of the first plurality of sequences comprises generating at least 10 variants.
(4) The method of any of the preceding items, wherein biological sequence data of the pathogen comprise a sequence of deoxyribonucleic acid, DNA, called genome, or a sequence of ribonucleic acid, RNA, called transcriptome, of said pathogen.
(5) The method of the preceding item, wherein a genome can be assembled from a plurality of short sequences, called reads, of different portions of the genome.
(6) The method of any of the preceding items, wherein sequence data input to the neural network comprise elements A, C, G, T of DNA or RNA encoded as one-hot representations 0001, 0010, 0100, and 1000.
(7) The method of any of the preceding items, wherein the resistances of a pathogen against the one or more anti-infectives are signified either by binary values from {0, 1} or by rational values from [0, 1], wherein 0 signifies no anti-infective resistance.
(8) The method of any of the preceding items, wherein the neural network, NN comprises one of: a convolutional neural network, CNN, a long short-term memory, LSTM, a recurrent neural network and a transformer type neural network.
(9) The method of the preceding item, wherein the neural network has one or more convolution layers and at least one fully connected layer.
(10) The method of the preceding item, wherein the each respective convolution layer has 128, 256, 512 or more kernels per layer with a shape of 200 by 4 per kernel.
(11) The method of any of the two preceding items, wherein each respective convolution layer applies dropout of 20% for training to avoid overfitting by randomly setting weights to zero.
(12) The method of any of the three preceding items, wherein the NN uses a cross entropy loss function for training, wherein the neural network, NN, has one or more max pooling layers, and a softmax classification layer to generate classification predictions.
(13) The method of any of the preceding items, wherein the neural network is trained on a plurality of specimen sequences with an Adam optimizer, and a learning rate of 0.00001 for preferably 146 epochs.
(14) A computer program product adapted to carry out the method as described in any one of the preceding items.
(15) A method for augmenting sequence data of a first plurality of biological sequences from a sample organism to train a neural network, NN, preferably the organism having a known property towards an external stimulus, to sequence data of a second plurality of variant biological sequences by generating sequence data of multiple variants of each sequence of the first plurality of sequences, preferably each variant carrying the same property towards the external stimulus as the corresponding sequence of the first plurality of sequences, wherein
   generating sequence data of multiple variants for each sequence of the first plurality of sequences, referred to as first sequence comprises:
   partitioning the first sequence into multiple parts, each part not exceeding a predetermined length; and
   generating multiple variants of the first sequence by concatenating at most once randomly selected parts of said multiple parts to respective variants of the first sequence, thereby obtaining sequence data of the second plurality of biological sequences.
(16) The method of item 15, wherein the sequence data is a sequence of nucleotides (nucleotide sequence) or a sequence of amino acids (amino acid sequence).
(17) The method of item 15 or 16, wherein said sample organism is a sample pathogen or sample subject.
(18) The method of item 15, 16 or 17, wherein said known property towards an external stimulus is resistance against one or more anti-infectives.
(19) The method of item 15, 16 or 17, wherein said known property towards an external stimulus is sensitivity against one or more anti-infectives.
(20) The method of any one of items 15 to 19, further comprising passing said augmented biological sequence data in lieu of the obtained biological sequence data to the input layer of a NN.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

As used herein the terms "about", "approximately" or "essentially" mean within 20%, preferably within 15%, preferably within 10%, and more preferably within 50 of a given value or range. It also includes the concrete number, i.e. "about 20" includes the number of 20.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

## Claims

1. A computer-implemented method for classifying resistances of a sample pathogen against one or more anti-infectives from biological sequence data of said sample pathogen, the biological sequence data comprising a sequence of deoxyribonucleic acid, DNA, called genome, or a sequence of ribonucleic acid, RNA, called transcriptome, of said pathogen, the method comprising:
augmenting sequence data of a first plurality of biological sequences of pathogens, different from the sample pathogen, having known resistances against the one or more anti-infectives, to sequence data of a second plurality of variant biological sequences by generating sequence data of multiple variants of each sequence of the first plurality of sequences, each variant carrying the same resistances against the one or more anti-infectives as the corresponding sequence of the first plurality of sequences, wherein
generating sequence data of multiple variants for each sequence of the first plurality of sequences, referred to as first sequence comprises:
partitioning the first sequence into multiple parts, each part not exceeding predetermined length; and
generating multiple variants of the first sequence by concatenating once randomly selected parts of said multiple parts to respective variants of the first sequence, thereby obtaining sequence data of the second plurality of biological sequences;
training a neural network, NN, via an input layer, specimen sequence data of the second plurality of biological sequences of pathogens labelled with known resistances of the respective pathogens, using forward propagation that repeatedly compares outputs of the neural network with corresponding ground truth labels to calculate an error using a loss function, and using backpropagation to calculate a gradient of the loss function to adjust the weighting values of the NN,
obtaining, after training the NN, biological sequence data for inferring resistances of a sample pathogen; and
passing said biological sequence data to an input layer of the NN with weights and biases after teaching incorporated;
generating, via an inference output layer of the NN, classification predictions of resistances of the sample pathogen against the one or more anti-infectives obtained from output of the neural network.

2. The method of claim 1, wherein passing said biological sequence data to the input layer of the NN further comprises:
generating augmented biological sequence data from the obtained biological sequence data by partitioning the obtained biological sequence data into multiple parts, each part not exceeding a predetermined length;
concatenating at most once randomly selected parts of said multiple parts to a variant of the obtained biological sequence data; and
passing the augmented biological sequence data in lieu of the obtained biological sequence data to the input layer of the NN.

3. The method of any one of the preceding claims, wherein generating sequence data of multiple variants of each sequence of the first plurality of sequences comprises generating at least 10 variants.

4. The method of any one of the preceding claims, wherein sequence data input to the neural network comprise elements A, C, G, T of DNA or RNA encoded as one-hot representations 0001, 0010, 0100, and 1000.

5. The method of any one of the preceding claims, wherein the resistances of a pathogen against the one or more anti-infectives are signified either by binary values from {0, 1} or by rational values from [0, 1], wherein 0 signifies no anti-infective resistance.

6. The method of any one of the preceding claims, wherein the neural network, NN comprises one of: a convolutional neural network, CNN, a long short-term memory, LSTM, a recurrent neural network and a transformer type neural network.

7. The method of claim 6, wherein the neural network has one or more convolution layers and at least one fully connected layer.

8. The method of claim 7, wherein each respective convolution layer applies dropout of 20% for training to avoid overfitting by randomly setting weights to zero.

9. The method of claim 7 or 8, wherein the NN uses a cross entropy loss function for training, wherein the neural network, NN, has one or more max pooling layers, and a softmax classification layer to generate classification predictions.

10. The method of any one of the preceding claims, wherein the neural network is trained on a plurality of specimen sequences with an Adam optimizer, and a learning rate of 0.00001 for preferably 146 epochs.

11. A computer program product adapted to carry out the method as claimed in any one of the preceding claims.

12. A computer implemented
method for augmenting sequence data of a first plurality of biological sequences from a sample organism to train a neural network, NN, preferably the organism having a known property towards an external stimulus, to sequence data of a second plurality of variant biological sequences by generating sequence data of multiple variants of each sequence of the first plurality of sequences, preferably each variant carrying the same property towards the external stimulus as the corresponding sequence of the first plurality of sequences, wherein the sequence data is a sequence of nucleotides (nucleotide sequence), wherein
generating sequence data of multiple variants for each sequence of the first plurality of sequences, referred to as first sequence comprises:
partitioning the first sequence into multiple parts, each part not exceeding a predetermined length; and
generating multiple variants of the first sequence by concatenating once randomly selected parts of said multiple parts to respective variants of the first sequence, thereby obtaining sequence data of the second plurality of biological sequences.

13. The method of claim 12, wherein said sample organism is a sample pathogen or sample subject.

14. The method of claim 12 or 13, wherein said known property towards an external stimulus is resistance against one or more anti-infectives, or sensitivity against one or more anti-infectives.

15. The method of any one of claims 12 to 14, further comprising passing said augmented biological sequence data in lieu of the obtained biological sequence data to the input layer of a NN.

## Patentansprüche

1. Computerimplementiertes Verfahren zum Klassifizieren von Resistenzen eines Probenpathogens gegen ein oder mehrere Antiinfektiva aus biologischen Sequenzdaten des Probenpathogens, wobei die biologischen Sequenzdaten eine Sequenz von Desoxyribonukleinsäure, DNA, genannt Genom, oder eine Sequenz von Ribonukleinsäure, RNA, genannt Transkriptom, des Pathogens umfasst, wobei das Verfahren umfasst:
Erweitern von Sequenzdaten einer ersten Vielzahl von biologischen Sequenzen von Pathogenen, die sich vom Probenpathogen unterscheiden und bekannte Resistenzen gegen das eine oder mehrere Antiinfektiva aufweisen, auf Sequenzdaten einer zweiten Vielzahl von varianten biologischen Sequenzen durch Erzeugen von Sequenzdaten vieler Varianten jeder Sequenz der ersten Vielzahl von Sequenzen, wobei jede Variante die gleichen Resistenzen gegen das eine oder die mehreren Antiinfektiva trägt wie die entsprechende Sequenz der ersten Vielzahl von Sequenzen, wobei
das Erzeugen von Sequenzdaten vieler Varianten für jede Sequenz der ersten Vielzahl von Sequenzen, die als erste Sequenz bezeichnet werden, umfasst:
Aufteilen der ersten Sequenz in mehrere Teile, wobei jeder Teil eine vorgegebene Länge nicht überschreitet; und
Erzeugen vieler Varianten der ersten Sequenz durch Verketten einmal zufällig ausgewählter Teile der mehreren Teile zu jeweiligen Varianten der ersten Sequenz, wodurch Sequenzdaten der zweiten Vielzahl von biologischen Sequenzen erhalten werden;
Trainieren eines neuronalen Netzes, NN, über eine Eingabeschicht, Speziessequenzdaten der zweiten Vielzahl von biologischen Sequenzen von Pathogenen, die mit bekannten Resistenzen der jeweiligen Pathogene markiert sind, unter Verwendung von Vorwärtspropagierung, die wiederholt Ausgaben des neuronalen Netzes mit entsprechenden Grundwahrheitsmarkierungen vergleicht, um unter Verwendung einer Verlustfunktion einen Fehler zu berechnen, und unter Verwendung von Rückwärtspropagierung, um einen Gradienten der Verlustfunktion zu berechnen, um die gewichteten Werte des NN anzupassen,
Erhalten von biologischen Sequenzdaten nach dem Training des NN, um Resistenzen eines Probenpathogens abzuleiten; und
Weitergeben der biologischen Sequenzdaten an eine Eingabeschicht des NN, wobei Gewichtungen und Bias-Werte nach dem Lernen integriert werden;
Erzeugen der Klassifizierungsvorhersagen von Resistenzen des Probenpathogens gegen das eine oder mehrere Antiinfektiva über eine Interferenzausgangsschicht des NN, erhalten aus der Ausgabe des neuronalen Netzes.

2. Verfahren nach Anspruch 1, wobei das Weitergeben der biologischen Sequenzdaten an die Eingabeschicht des NN ferner umfasst:
Erzeugen erweiterter biologischer Sequenzdaten aus den erhaltenen biologischen Sequenzdaten durch Aufteilen der erhaltenen biologischen Sequenzdaten in viele Teile, wobei jeder Teil eine vorgegebene Länge nicht überschreitet;
Verketten von höchstens einmal zufällig ausgewählten Teilen der vielen Teile zu einer Variante der erhaltenen biologischen Sequenzdaten; und
Weitergeben der erweiterten biologischen Sequenzdaten anstelle der erhaltenen biologischen Sequenzdaten an die Eingabeschicht des NN.

3. Verfahren nach einem der vorhergehenden Ansprüche, wobei das Erzeugen von Sequenzdaten von vielen Varianten jeder Sequenz der ersten Vielzahl von Sequenzen das Erzeugen von mindestens 10 Varianten umfasst.

4. Verfahren nach einem der vorhergehenden Ansprüche, wobei die in das neuronale Netz eingegebenen Sequenzdaten die Elemente A, C, G, T von DNA oder RNA umfassen, die als One-Hot-Darstellungen 0001, 0010, 0100 und 1000 codiert sind.

5. Verfahren nach einem der vorhergehenden Ansprüche, wobei die Resistenzen eines Pathogens gegen das eine oder mehrere Antiinfektiva entweder durch binäre Werte aus {0, 1} oder durch rationale Werte aus [0, 1] gekennzeichnet werden, wobei 0 keine Antiinfektiva-Resistenz bedeutet.

6. Verfahren nach einem der vorhergehenden Ansprüche, wobei das neuronale Netz NN eines umfasst aus: einem konvolutionalen neuronalen Netz CNN, einem langen Kurzzeitgedächtnis LSTM, einem rekurrenten neuronalen Netz und einem neuronalen Netz vom Transformertyp.

7. Verfahren nach Anspruch 6, wobei das neuronale Netz eine oder mehrere Faltungsschichten und mindestens eine vollständig verbundene Schicht aufweist.

8. Verfahren nach Anspruch 7, wobei jede entsprechende Faltungsschicht beim Training einen Dropout von 20 % durch zufälliges Setzen der Gewichte auf Null anwendet, um eine Überanpassung zu vermeiden.

9. Verfahren nach Anspruch 7 oder 8, wobei das NN eine Kreuzentropie-Verlustfunktion zum Trainieren nutzt, wobei das neuronale Netz NN eine oder mehrere Max-Pooling-Schichten und eine Softmax-Klassifizierungsschicht aufweist, um Klassifizierungsvorhersagen zu erzeugen.

10. Verfahren nach einem der vorhergehenden Ansprüche, wobei das neuronale Netz mit einem Adam-Optimierer an einer Vielzahl von Speziessequenzen und einer Lernrate von 0,00001 über vorzugsweise 146 Epochen trainiert wird.

11. Computerprogrammprodukt, angepasst zum Ausführen des Verfahrens nach einem der vorhergehenden Ansprüche.

12. Computerimplementiertes Verfahren zum Erweitern von Sequenzdaten einer ersten Vielzahl von biologischen Sequenzen von einem Probenorganismus zum Trainieren eines neuronalen Netzes NN, wobei der Organismus vorzugsweise eine bekannte Eigenschaft gegen den externen Stimulus aufweist, auf Sequenzdaten einer zweiten Vielzahl varianter biologischer Sequenzen durch Erzeugen von Sequenzdaten vieler Varianten von jeder Sequenz der ersten Vielzahl von Sequenzen, wobei jede Variante vorzugsweise die gleiche Eigenschaft gegen den externen Stimulus trägt wie die entsprechende Sequenz der ersten Vielzahl von Sequenzen, wobei die Sequenzdaten eine Sequenz von Nukleotiden (Nukleotidsequenz) sind, wobei
das Erzeugen von Sequenzdaten vieler Varianten für jede Sequenz der ersten Vielzahl von Sequenzen, die als erste Sequenz bezeichnet werden, umfasst:
Aufteilen der ersten Sequenz in mehrere Teile, wobei jeder Teil eine vorgegebene Länge nicht überschreitet; und
Erzeugen vieler Varianten der ersten Sequenz durch Verketten einmal zufällig ausgewählter Teile der mehreren Teile zu jeweiligen Varianten der ersten Sequenz, wodurch Sequenzdaten der zweiten Vielzahl von biologischen Sequenzen erhalten werden.

13. Verfahren nach Anspruch 12, wobei der Probenorganismus ein Probenpathogen oder ein Probensubjekt ist.

14. Verfahren nach Anspruch 12 oder 13, wobei die bekannte Eigenschaft gegen einen externen Stimulus die Resistenz gegen ein oder mehrere Antiinfektiva oder die Empfindlichkeit gegen ein oder mehrere Antiinfektiva ist.

15. Verfahren nach einem der Ansprüche 12 bis 14, ferner umfassend das Weitergeben der erweiterten biologischen Sequenzdaten anstelle der erhaltenen biologischen Sequenzdaten an die Eingabeschicht eines NN.

## Revendications

1. Procédé mis en œuvre par ordinateur pour classer les résistances d'un pathogène échantillon à un ou plusieurs anti-infectieux à partir de données de séquence biologique dudit pathogène échantillon, les données de séquence biologique comprenant une séquence d'acide désoxyribonucléique, ADN, appelée génome, ou une séquence d'acide ribonucléique, ARN, appelée transcriptome, dudit pathogène, le procédé comprenant :
l'augmentation des données de séquence d'une première pluralité de séquences biologiques de pathogènes, différents du pathogène échantillon, ayant des résistances connues aux un ou plusieurs anti-infectieux, aux données de séquence d'une deuxième pluralité de séquences biologiques variantes en générant des données de séquence de multiples variantes de chaque séquence de la première pluralité de séquences, chaque variante portant les mêmes résistances aux un ou plusieurs anti-infectieux que la séquence correspondante de la première pluralité de séquences, où
la génération de données de séquence de multiples variantes pour chaque séquence de la première pluralité de séquences, appelées première séquence, comprend :
la division de la première séquence en multiples parties, chaque partie ne dépassant pas une longueur prédéterminée ; et
la génération de multiples variantes de la première séquence en concaténant une fois des parties sélectionnées de manière aléatoire desdites multiples parties à des variantes respectives de la première séquence, obtenant ainsi des données de séquence de la deuxième pluralité de séquences biologiques ;
l'entraînement d'un réseau neuronal, RN, via une couche d'entrée, des données de séquence d'échantillons de la deuxième pluralité de séquences biologiques de pathogènes étiquetées avec des résistances connues des pathogènes respectifs, en utilisant une propagation directe qui compare de manière répétée les sorties du réseau neuronal avec des étiquettes de vérité de terrain correspondantes afin de calculer une erreur à l'aide d'une fonction de perte, et en utilisant une rétropropagation pour calculer un gradient de la fonction de perte afin d'ajuster les valeurs de pondération du RN,
l'obtention, après l'entraînement du RN, de données de séquence biologique permettant de déduire les résistances d'un pathogène échantillon ; et
la transmission desdites données de séquence biologique à une couche d'entrée du RN avec des poids et des biais après apprentissage intégrés ;
la génération, via une couche de sortie d'inférence du RN, de prédictions de classification de résistances du pathogène échantillon aux un ou plusieurs anti-infectieux obtenues à partir de la sortie du réseau neuronal.

2. Procédé selon la revendication 1, dans lequel la transmission desdites données de séquence biologique à la couche d'entrée du RN comprend en outre :
la génération de données de séquence biologique augmentées à partir des données de séquence biologique obtenues en divisant les données de séquence biologique obtenues en plusieurs parties, chaque partie ne dépassant pas une longueur prédéterminée ;
la concaténation au plus une fois de parties sélectionnées de manière aléatoire parmi lesdites parties multiples à une variante des données de séquence biologique obtenues ; et
la transmission des données de séquence biologique augmentées à la place des données de séquence biologique obtenues à la couche d'entrée du RN.

3. Procédé selon l'une quelconque des revendications précédentes, dans lequel la génération de données de séquence de multiples variantes de chaque séquence de la première pluralité de séquences comprend la génération d'au moins 10 variantes.

4. Procédé selon l'une quelconque des revendications précédentes, dans lequel les données de séquence entrées dans le réseau neuronal comprennent les éléments A, C, G, T de l'ADN ou de l'ARN codés sous forme de représentations one-hot 0001, 0010, 0100 et 1000.

5. Procédé selon l'une quelconque des revendications précédentes, dans lequel les résistances d'un pathogène aux un ou plusieurs anti-infectieux sont représentées soit par des valeurs binaires valant {0, 1}, soit par des valeurs rationnelles valant [0, 1], où 0 signifie l'absence de résistance aux anti-infectieux.

6. Procédé selon l'une quelconque des revendications précédentes, dans lequel le réseau neuronal RN comprend l'un des éléments suivants : un réseau neuronal convolutif RNC, une mémoire à long et court terme LSTM, un réseau neuronal récurrent et un réseau neuronal de type transformateur.

7. Procédé selon la revendication 6, dans lequel le réseau neuronal comporte une ou plusieurs couches de convolution et au moins une couche entièrement connectée.

8. Procédé selon la revendication 7, dans lequel chaque couche de convolution respective applique un abandon aléatoire de 20 % pour l'apprentissage afin d'éviter le surapprentissage en mettant aléatoirement les poids à zéro.

9. Procédé selon la revendication 7 ou 8, dans lequel le réseau neuronal utilise une fonction de perte d'entropie croisée pour l'apprentissage, dans lequel le réseau neuronal, RN, comporte une ou plusieurs couches de regroupement maximal et une couche de classification softmax pour générer des prédictions de classification.

10. Procédé selon l'une quelconque des revendications précédentes, dans lequel le réseau neuronal est entraîné sur une pluralité de séquences d'échantillons avec un optimiseur Adam et un taux d'apprentissage de 0,00001 pendant de préférence 146 époques.

11. Produit logiciel destiné à mettre en œuvre le procédé selon l'une quelconque des revendications précédentes.

12. Procédé mis en œuvre par ordinateur pour augmenter des données de séquence d'une première pluralité de séquences biologiques provenant d'un organisme échantillon afin d'entraîner un réseau neuronal, RN, de préférence l'organisme ayant une propriété connue vis-à-vis d'un stimulus externe, à des données de séquence d'une deuxième pluralité de séquences biologiques variantes en générant des données de séquence de multiples variantes de chaque séquence de la première pluralité de séquences, de préférence chaque variante portant la même propriété vis-à-vis du stimulus externe que la séquence correspondante de la première pluralité de séquences, où les données de séquence sont une séquence de nucléotides (séquence nucléotidique), où
la génération de données de séquence de multiples variantes pour chaque séquence de la première pluralité de séquences, appelées première séquence, comprend :
la division de la première séquence en plusieurs parties, chacune ne dépassant pas une longueur prédéterminée ; et
la génération de plusieurs variantes de la première séquence en concaténant une fois des parties sélectionnées de manière aléatoire desdites plusieurs parties à des variantes respectives de la première séquence, obtenant ainsi des données de séquence de la deuxième pluralité de séquences biologiques.

13. Procédé selon la revendication 12, dans lequel ledit organisme échantillon est un pathogène échantillon ou un sujet échantillon.

14. Procédé selon la revendication 12 ou 13, dans lequel ladite propriété connue vis-à-vis d'un stimulus externe est une résistance à un ou plusieurs anti-infectieux ou une sensibilité à un ou plusieurs anti-infectieux.

15. Procédé selon l'une quelconque des revendications 12 à 14, comprenant en outre le passage desdites données de séquence biologique augmentées à la place des données de séquence biologique obtenues à la couche d'entrée d'un RN.
